# EUROPEAN PATENT APPLICATION

(11) **EP 4 417 192 A1**
(43) Date of publication of application: **21.08.2024**
(21) Application number: 23157328.8
(22) Date of filing: 17.02.2023
(51) Int. Cl.: A61K 9/00

(54) **MEDICAL DEVICE, KIT, METHOD, AND ANTI-INFLAMMATORY SUBSTANCE**

(71) Applicant: meliodys medical UG, 80333 München (DE)
(72) Inventor: WOLF, Simone, 80333 München (DE)
(74) Representative: Vossius & Partner Patentanwälte Rechtsanwälte mbB

(57) **Abstract**

The present invention relates to a medical device (10) configured to be deposited at least partially within a patient's body via the patient's vagina for treating dysmenorrhea and/or endometriosis. The medical device (10) includes at least one carrier (12), at least one releasing element (14), which is at least partially attached to the carrier (12) and is made of at least one biodegradable material, and at least one anti-inflammatory substance (16) which is embedded in the releasing element (14) such that the anti-inflammatory substance (16) is releasable within the patient's body, when the medical device (10) is deposited within the patient's body.

The present invention further relates to a kit, a method, and an anti-inflammatory substance.

## Description

There are several known ailments relating to a female reproductive system, many of which may cause discomfort, in particular severe discomfort at least in some cases. For instance, dysmenorrhea and endometriosis are known disorders of the female reproductive system which can range from mild to severe cases.

Dysmenorrhea, also known as perimenstrual or menstrual pain, is an ailment which affects up to 81% of women during their reproductive life span, as estimated by the World Health Organization (WHO). From a clinical perspective, dysmenorrhea can be grouped into primary dysmenorrhea and secondary dysmenorrhea. Primary dysmenorrhea, which is the most common form of dysmenorrhea, has no apparent cause, such as an underlying anatomical disorder. Instead, primary dysmenorrhea seems to be based on interacting genetic, epigenetic and environmental factors. Secondary dysmenorrhea, on the other hand, is caused by identifiable underlying diseases, of which endometriosis is the most common. Hence, endometriosis may exacerbate dysmenorrhea and worsen the symptoms of the affected woman.

The detailed mechanisms of dysmenorrhea are still not fully understood (see Ferries-Rowe et al. (2020): Primary Dysmenorrhea: Diagnosis and Therapy). It is clear, however, that abnormal prostaglandin synthesis and prostaglandin signaling are at the center of the disorder. In the affected women, hormonal changes, i.e., the normal drop of progesterone shortly before menstruation, causes increased production of inflammatory mediators, e.g., prostaglandins. Prostaglandins may cause constriction of small uterine blood vessels leading to tissue ischemia and discomfort, e.g., pain. In addition, prostaglandins are directly involved in pain signaling. While some external factors, such as a history of childbearing, nutrition, and physical activity, may affect uterine prostaglandin signaling and therefore pain generation, the occurrence of primary dysmenorrhea is most likely determined by congenital and genetic factors.

Endometriosis is the most common cause of secondary dysmenorrhea and affects roughly 6-10% of all women. The hallmark of endometriosis is the presence of endometrium (the lining of the uterine cavity) in other regions of the affected woman's body outside of the uterine cavity. Most importantly, however, endometriosis is characterized by exacerbated chronic cyclical inflammation of the uterus and other pelvic tissues. This inflammation is even more pronounced than in primary dysmenorrhea. The chronically recurring inflammatory episodes during each menstruation can lead to progressive tissue destruction and fibrosis.

The severity of dysmenorrhea, whether primary dysmenorrhea or secondary dysmenorrhea, may range from mild pelvic discomfort to incapacitating pain which occurs in up to 29% of all women. In a recent large survey involving 42,879 women, 85% of the participants reported to have abdominal pain during their menstrual period (see Schoep et al., The impact of menstrual symptoms on everyday life: a survey among 42,879 women, Am J Obstet Gynecol, 220(6):569, doi: 10.1016/j.ajog.2019.02.048). In the US, it has been estimated that 10-30% of all working or studying women with dysmenorrhea lose 1 to 2 working/studying days per month due to dysmenorrhea which amounts to an annual loss of about 600 million working hours resulting in a monetary loss of roughly 2 billion USD per year (see Ferries-Rowe et al., Primary Dysmenorrhea: Diagnosis and Therapy, Obstet Gynecol, 136(5):1047-1058, doi: 10.1097/AOG.0000000000004096). Hence, the burden caused by dysmenorrhea, both on each individual woman and on the economy, is considerable. Moreover, currently, there is no cure for dysmenorrhea. Thus, improving care for affected women should be a major public health priority.

Though treatments for dysmenorrhea and/or endometriosis are available, the treatments known from the prior art have several drawbacks. In particular, the effectiveness of the treatments known from the prior art may be inadequate in relieving the discomfort caused by dysmenorrhea and/or endometriosis. The result of inadequate treatment may be severe since it may lead to chronic pain. In addition, several of the known treatments are prescribed and/or used periodically on an "as needed" basis, i.e., when symptoms of dysmenorrhea are acute or imminent, which forces the subject to frequently actively seek treatment, or at least actively take one or more countermeasures, when symptoms of dysmenorrhea are acute or imminent, which makes the treatment more cumbersome for the affected women. This may also limit the effectiveness of the treatment. In addition, several of the known treatments may cause one or more side effects, in particular one or more relatively severe side effects. The prior art relating to the management of dysmenorrhea includes hormonal treatments which interfere significantly with many organ systems, frequently causing unacceptable adverse effects. The other mainstay of the prior art in the management of dysmenorrhea is the systemic administration (e.g., the administration of pills taken by mouth) of anti-inflammatory drugs. The systemic administration of these drugs also causes frequent adverse effects in non-reproductive organs, such as the gastrointestinal tract and/or the kidneys. During systemic drug administration, these organs are exposed to high concentrations of the active anti-inflammatory drug, while only a fraction of the active drug provides an effect on the target organs of the reproductive tract.

These drawbacks have not, or at least not sufficiently, been addressed in the prior art.

It is therefore an object of the invention to provide an improved means of treatment for treating dysmenorrhea and/or endometriosis, in particular a means of treatment which addresses one or more of the above-identified disadvantages.

This objective is achieved by a medical device according to a first aspect of the present disclosure, as defined by the features of claim 1. Preferred embodiments are defined by the features of the dependent claims, respectively.

The medical device may be configured to be deposited at least partially within a patient's body via the patient's vagina for treating dysmenorrhea and/or endometriosis.

The medical device may include at least one carrier and at least one releasing element. The releasing element may be made of at least one biodegradable material, preferably at least one biodegradable biopolymer. The medical device may further include at least one anti-inflammatory substance. The anti-inflammatory substance may be embedded and/or trapped in the releasing element. Alternatively, the anti-inflammatory substance may be incorporated into the medical device in a variety of alternative manners. For instance, the anti-inflammatory substance may be attached directly to the carrier, in which case the releasing element, as an intermediate structure connecting the carrier and the anti-inflammatory substance, may be omitted. Alternatively, or additionally, the anti-inflammatory substance may be coated on a surface, preferably an outer surface, of the releasing element and/or a surface, preferably an outer surface, of the carrier.

The releasing element may be at least partially attached to the carrier such that the anti-inflammatory substance is releasable within the patient's body, when the medical device is deposited within the patient's body. The anti-inflammatory substance may prevent, or at least reduce, the production of inflammatory agents, such as prostaglandins, and/or may prevent, or at least reduce, pain signaling caused by inflammatory agents, such as prostaglandins. This may reduce pain and/or discomfort of the patient caused by dysmenorrhea and/or endometriosis.

Providing a medical device which includes the anti-inflammatory substance and is deployable into the patient's body via the patient's vagina, e.g., into the patient's uterus, more specifically the patient's uterine cavity, may allow the anti-inflammatory substance to be released and applied primarily locally to one or more female reproductive organs, in particular within the patient's uterus, in particular at or at least proximate to one or more regions within the patient's body which are affected by the ailment.

Such a local release and application of the anti-inflammatory substance may reduce the systemic adverse effects of the anti-inflammatory substance, e.g., compared with an oral ingestion and/or injection of a treatment substance. This may also increase the effectiveness and/or efficiency of the anti-inflammatory substance in providing relief against symptoms of dysmenorrhea and/or endometriosis, e.g., compared with oral ingestion and/or injection of a treatment substance.

Moreover, since the *continuous* release of the anti-inflammatory substance may locally, i.e., at the site of discomfort and/or pain, prevent, or at least limit, the production of inflammatory mediators, e.g., prostaglandins, in the first place, the dose of the anti-inflammatory substance may be reduced, e.g., compared with oral ingestion and/or injection of a treatment substance, when inflammatory mediators have already built up and cause symptoms. This may reduce adverse effects, or at least the risk thereof, caused by the anti-inflammatory substance. Moreover, since the known anti-inflammatory treatments which are prescribed and/or used periodically, e.g., on an "as needed" basis, are generally taken once the pain and/or discomfort has already occurred, such treatments may be less effective, at least based on a similar or same dose thereof, than the treatment provided by the medical device described herein.

Furthermore, the medical device may continuously, or at least for certain time periods, release the anti-inflammatory substance within the patient's body, while the medical device remains lodged within the patient's body. Hence, the patient is not required to re-administer the medical device, at least for an extended period of time, e.g., for at least 6 or 12 months, preferably for at least 24 months. Hence, in particular compared with the known treatments which are prescribed and/or used periodically, e.g., on an "as needed" basis, the medical device described herein may provide a more reliable, convenient, patient-friendly and/or effective treatment for treating dysmenorrhea and/or endometriosis. In particular, it has been found that pain control, in particular of dysmenorrhea and/or endometriosis, in particular when using anti-inflammatory substances, may be more effective and/or more efficient when taken anticipatory, i.e., before the onset of the pain and/or discomfort.

Hormonal-based contraceptives, such as oral contraceptive pills or hormone releasing intrauterine devices (IUD) are commonly used to treat dysmenorrhea which may, however, cause one or more side effects, sometimes severe side effects. For instance, estrogen and progestins, which are primarily used, may cause side effects including, but not limited to, mood changes, including depression, weight gain and thrombosis. Therefore, some of these treatments are contraindicated in many women, such as overweight patients, smokers, patients with a history of thrombosis, arterial hypertension, liver disease, migraine, and others. Hence, providing a medical device which includes an anti-inflammatory substance may reduce the side effects, or at least reduce the risk of side effects, of the treatment, e.g., compared with hormonal-based contraceptives.

The releasing element and the carrier may be attached to each other by any means suitable to provide a desired degree of attachment between the releasing element and the carrier. The releasing element and the carrier may be attached to each other in a fixed manner, i.e., such that the releasing element and the carrier are immovable relative to each other. Alternatively, the attachment between the releasing element and the carrier may allow some movement between the releasing element and the carrier.

The releasing element and the carrier may be permanently attached to each other, i.e., such that the releasing element may not be removed from the carrier without compromising the structural integrity of the carrier and the releasing element.

Alternatively, the releasing element and the carrier may be removably attached to each other. For instance, this may enable the carrier and the releasing element to be assembled and disassembled, preferably a plurality of times. This may allow, e.g., the releasing element and the carrier to be provided as a kit which is assembled, e.g., by the user and/or medical personnel. This may allow, for instance, the carrier to be configured as a reusable part which may be reloaded with one or more replacement releasing elements, e.g., once the anti-inflammatory substance of the original releasing element has been fully consumed. Moreover, this may allow the carrier to be more easily combined with a plurality of different releasing elements and/or vice versa, e.g., to adapt to different patient anatomies, such as by providing a plurality of different releasing elements and/or a plurality of different carriers which have different shapes, sizes, materials, etc. Moreover, this may allow a plurality of releasing elements which have different anti-inflammatory substances which differ in one or more properties, respectively, e.g., in the amount and/or weight of the anti-inflammatory substance, the type of anti-inflammatory substance, a release rate of the anti-inflammatory substance, etc., to be combined with one or more carriers.

The medical device may be configured to remain securely deposited within the patient's body, preferably within the patient's uterus, more specifically the patient's uterine cavity, for an extended period of time, including during daily activities, such as exercise. To provide a secure hold of the medical device within the patient's body, the medial device, e.g., the carrier and/or the releasing element, may include one or more securing mechanisms, e.g., one or more engagement arms, configured to engage with at least a portion of a patient's anatomy within the patient's body to secure the medical device within the patient's body.

The carrier may be made of a material which is non-resorbable and/or non-absorbable and/or non-degradable by the patient's body. Preferably, the carrier is made of plastic, e.g., low-density polyethylene (LDPE).

Preferably, the at least one anti-inflammatory substance includes at least one non-hormonal and/or non-steroidal anti-inflammatory substance, preferably celecoxib and/or diclofenac. However, a number of alternative anti-inflammatory substances may also be employed. For instance, alternative anti-inflammatory substances may include one or more of ibuprofen, ketoprofen, mefenamic acid, naproxen, piroxicam, and indomethacin. Alternative non-hormonal anti-inflammatory substances may include glyceryl trinitrate which may require even lower concentrations than other anti-inflammatory substances, in particular non-hormonal anti-inflammatory substances.

Preferably, the medical device is configured to be deposited in at least a section of the patient's uterus, more specifically the patient's uterine cavity. In other words, the medical device may be configured as an intrauterine device. This may allow a more targeted and localized release and application of the anti-inflammatory substance, i.e., within the patient's uterus, to more precisely target the origin of discomfort and/or pain caused by dysmenorrhea and/or endometriosis.

Preferably, the medical device is free of hormonal substances. This may prevent side effects caused by hormonal substances, e.g., compared with hormonal-based contraceptives, such as oral contraceptive pills or hormone releasing intrauterine devices (IUD), as discussed above.

Preferably, the medical device is configured to release the anti-inflammatory substance continuously at a rate larger than zero within the patient's body, when the medical device is deposited within the patient's body. This may allow the medical device to continuously release the anti-inflammatory substance within the patient's body, while the medical device remains lodged within the patient's body, which may provide a more effective and/or efficient relief of discomfort and/or pain to the patient, as described above.

Preferably, the releasing element is configured to interact with an environment within the patient's body to trigger the release of the anti-inflammatory substance. Preferably, the releasing element is configured to continuously release the anti-inflammatory substance thereafter. For instance, the release of the anti-inflammatory substance may be activated by an aqueous and/or warm environment within the patient's body.

Preferably, the releasing element is configured to at least partially disintegrate within the patient's body, when the medical device is deposited within the patient's body. The anti-inflammatory substance may be at least partially embedded in the releasing element such that the anti-inflammatory substance is released within the patient's body as the releasing element disintegrates, when the medical device is deposited within the patient's body. Alternatively, the releasing element may not disintegrate, at least not in order to release the anti-inflammatory substance. Instead, the anti-inflammatory substance may be applied to a portion of the carrier and/or the releasing element, e.g., as at least one coating, and the anti-inflammatory substance may be released/discharged from the releasing element.

Preferably, the anti-inflammatory substance is dispersed within the releasing element.

Preferably, the releasing element is configured to at least partially degrade within the patient's body at a preferably constant rate, when the medical device is deposited within the patient's body, such that the anti-inflammatory substance is released into the patient's body as the releasing element degrades.

Preferably, the releasing element, including the anti-inflammatory substance, is configured as a solid component. The releasing element may be at least partially hollow. The releasing element may be configured as a tube configured to at least partially surround at least a section of the carrier. The term "solid" refers to an aggregate state of the releasing element, including the anti-inflammatory substance. Providing the releasing element, including the anti-inflammatory substance, in a solid aggregate state may facilitate administering the medical device into the patient's body, e.g., by providing a sturdier structure to the medical device. Moreover, this may allow the release of the anti-inflammatory substance to be controlled more precisely, e.g., which may allow the dose and/or release rate of the anti-inflammatory substance to be reduced.

Preferably, the carrier includes at least one receiving section which is configured to securely receive the releasing element. The receiving section may be configured as an elongated member which is configured to be received at least partially within the releasing element or vice versa. Preferably, the releasing element includes at least one lumen configured to at least partially receive the receiving section of the carrier or vice versa.

Preferably, the receiving section has a diameter of no more than 2.4 mm, more preferably no more than 2.2 mm, more preferably no more than 2 mm, more preferably no more than 1.8 mm, more preferably no more than 1.6 mm, more preferably no more than 1.4 mm, more preferably no more than 1.2 mm, more preferably no more than 1 mm. This may limit the size of the carrier, and thus of the medical device, which may reduce discomfort which may be caused by the medical device to the patient. The diameter, i.e., the width, of the receiving section may be configured to be relatively small, e.g., according to the above-identified values, since the releasing element may be configured to provide stability to the carrier at the receiving section, e.g., by surrounding and/or extending along at least a section of the receiving section. In fact, the releasing element may replace the receiving section partially or entirely.

Preferably, the medical device includes at least one securing mechanism configured to secure the medical device in at least a section of the patient's uterus, more specifically the patient's uterine cavity. The securing mechanism may be configured in any manner which is suitable to engage with at least a section of the patient's uterus to secure the medical device in at least a section of the patient's uterus. The securing mechanism may be provided by the carrier. Preferably, the carrier, including the securing mechanism, may be formed integrally, Alternatively, the carrier may include a plurality of assembled parts. Alternatively, or additionally, the securing mechanism may be provided by the releasing element and/or by one or more further components of the medical device.

Preferably, the securing mechanism includes at least one arm which is configured to engage with a wall of the patient's uterus to secure the medical device within the patient's uterus.

The arm may be provided by the carrier. For instance, the arm may extend from at least one main section of the carrier, e.g., from the receiving section. Preferably, the receiving section and the arm may be formed integrally. Alternatively, the receiving section and the arm may be configured as separate components which are assembled. The arm may be flexible, e.g., bendable and/or stretchable, preferably elastically deformable, e.g., to facilitate deployment of the medical device into the patient's body and/or to facilitate securing the medical device within the patient's body.

Preferably, the securing mechanism includes at least two arms which are configured to engage with substantially opposite sides of the patient's uterus to secure the medical device within the patient's uterus. This may further decrease the risk of a displacement and/or a dislodging of the medical device from the intended location of the medical device in the patient's uterus. The arms may be flexible, e.g., bendable and/or stretchable, preferably elastically deformable.

Preferably, the arms are attached to the receiving section such that the arms and the receiving section are configured substantially in a Y-form or a T-form. This may provide a shape which corresponds, or is at least adapted, to the general shape of a woman's uterine cavity to facilitate secure placement of the medical device in the patient's cavity.

Preferably, the releasing element including the embedded anti-inflammatory substance is at least partially attached to at least one of the arms, preferably both arms. This may allow an area of a releasing surface of the medical device, from which the anti-inflammatory substance may be released, to be tailored to the desired needs, e.g., by increasing the area of the releasing surface of the medical device, e.g., to provide a more effective and/or efficient treatment.

Preferably, the medical device is configured to release the anti-inflammatory substance, more preferably continuously, for at least 10 months, more preferably at least 11 months, more preferably at least 12 months, more preferably at least 13 months, more preferably at least 14 months, more preferably at least 15 months, more preferably at least 16 months, more preferably at least 17 months, more preferably at least 18 months, more preferably at least 19 months, more preferably at least 20 months, more preferably at least 21 months, more preferably at least 22 months, more preferably at least 23 months, more preferably at least 24 months within the patient's body. The above-identified time periods may allow the patient to use a single medical device for an extended period of time, i.e., for the above-identified time periods, without having to re-administer a further medical device during the respective time period. This may provide a more reliable, convenient, patient-friendly and/or effective treatment for treating dysmenorrhea and/or endometriosis.

Preferably, the medical device is configured to release the anti-inflammatory substance, more preferably continuously, at a daily release rate of less than 3 mg per day, more preferably less than 2.8 mg per day, more preferably less than 2.6 mg per day, more preferably less than 2.4 mg per day, more preferably less than 2.2 mg per day, more preferably less than 2 mg per day, more preferably less than 1.8 mg per day, more preferably less than 1.6 mg per day, more preferably less than 1.4 mg per day, more preferably less than 1.2 mg per day, more preferably less than 1 mg per day. Limiting the daily release rate per the above-identified values may allow the release of the anti-inflammatory substance to be controlled more precisely and/or to reduce an exposure of the patient to the anti-inflammatory substance per time unit, e.g., compared with the known treatments which are prescribed and/or used periodically on an "as needed" basis. This may reduce potential side effects and/or toxicity of the anti-inflammatory substance.

Preferably, the medical device is configured to release the anti-inflammatory substance at a daily release rate in a range from 0.2 mg to 1 mg per day, more preferably from 0.4 mg to 1 mg per day, more preferably from 0.6 mg to 1 mg per day, more preferably from 0.8 mg to 1 mg per day.

Preferably, the medical device is configured to release the anti-inflammatory substance at a daily release rate with a day-to-day variance of no more than 0.3 mg per day, more preferably no more than 0.2 mg, more preferably no more than 0.1 mg. This may allow the release of the anti-inflammatory substance to be controlled more precisely which may reduce potential side effects of the anti-inflammatory substance and/or may increase the effectiveness and/or efficiency of the treatment via the medical device described herein.

Preferably, the medical device is provided with at least 100 mg, more preferably at least 150 mg, more preferably at least 200 mg, more preferably at least 250 mg, more preferably at least 300 mg, more preferably at least 350 mg, more preferably at least 400 mg, more preferably at least 450 mg, more preferably at least 500 mg of the anti-inflammatory substance. It may be desired to strike a balance between the weight of the medical device, which may be desired to be relatively low, and the lifetime and/or effectiveness of the anti-inflammatory substance, e.g., by choosing an appropriate/desired amount/weight of the anti-inflammatory substance. Hence, the weight of the anti-inflammatory substance may be chosen accordingly, e.g., according to the above-identified value(s).

Preferably, the medical device further includes a withdrawal apparatus configured to enable the medical device to be withdrawn from the patient's body. The withdrawal apparatus may be configured as one or more graspable elements which can be grasped by a human, e.g., the patient and/or medical personnel. The withdrawal apparatus may allow a safe and secure retrieval of the medical device from the patient's body. For instance, the withdrawal apparatus may include one or more elongated elements, e.g., one or more strings, which extend towards or into the patient's cervical canal and/or vagina, when the medical device is employed in an operational state within the patient's body. The withdrawal apparatus is preferably made of a material which is non-resorbable and/or non-absorbable and/or non-degradable by the patient's body.

Preferably, the medical device further includes at least one hemostatic agent, which is preferably attached to the carrier. This may stop, or at least reduce, bleeding which may occur at or near a site of deployment of the medical device in the patient's body, e.g., due to a potential interaction between the anatomy of the patient's body and the medical device, and/or due to increased menstrual bleeding.

The object mentioned at the beginning is also solved by a kit according to a second aspect of the present disclosure, as defined by the features of claim 13. The features, embodiments, and advantages, as described above with respect to the medical device according to the first aspect of the present disclosure, also apply to the kit accordingly.

The kit may include at least one carrier configured to be deposited at least partially within a patient's body via the patient's vagina and at least one releasing element. The releasing element may be made of at least one biodegradable material.

The kit may further include at least one anti-inflammatory substance. The anti-inflammatory substance may be embedded in the releasing element.

The releasing element may be configured to be at least partially attachable to the carrier such that the anti-inflammatory substance is releasable within the patient's body, when the releasing element is attached to the carrier and the carrier including the releasing element is deposited within the patient's body.

The object mentioned at the beginning is also solved by a method for treating at least one ailment of a female reproductive system of a patient, preferably dysmenorrhea and/or endometriosis by means of a medical device, preferably by means of the above described medical device. The features, embodiments, and advantages, as described above with respect to the medical device according to the first aspect of the present disclosure, also apply to the method accordingly.

The method is configured for treating at least one ailment of a female reproductive system of a patient, preferably dysmenorrhea and/or endometriosis, preferably by means of the medical device according to any of the embodiments described herein.

The method may include depositing a medical device which includes at least one carrier, at least one releasing element made of at least one biodegradable material and at least one anti-inflammatory substance embedded in the releasing element at least partially within the patient's body via the patient's vagina.

The method may further include releasing the anti-inflammatory substance within the patient's body, when the medical device has been deposited within the patient's body.

The object mentioned at the beginning is also solved by an anti-inflammatory substance according to a fourth aspect of the present disclosure, as defined by the features of claim 14. The features, embodiments, and advantages, as described above with respect to the medical device according to the first aspect of the present disclosure, also apply to the anti-inflammatory substance accordingly.

The anti-inflammatory substance is preferably a non-steroidal anti-inflammatory substance and/or a non-hormonal anti-inflammatory substance. The anti-inflammatory substance may be diclofenac and/or celecoxib. However, a number of alternative anti-inflammatory substances may also be employed. For instance, alternative anti-inflammatory substances may include one or more of ibuprofen, ketoprofen, mefenamic acid, naproxen, piroxicam, and indomethacin. Alternative (non-hormonal) anti-inflammatory substances may include glyceryl trinitrate which may require even lower concentrations than other anti-inflammatory substances, in particular non-hormonal anti-inflammatory substances. The anti-inflammatory substance may be used in treating dysmenorrhea and/or endometriosis, preferably by means of the medical device according to any of the embodiments described herein.

The anti-inflammatory substance may be embedded in at least one releasing element made of at least one biodegradable material. The releasing element may be at least partially attached to at least one carrier. The carrier, including the releasing element and the embedded anti-inflammatory substance, may be deposited at least partially within the patient's body via the patient's vagina. The anti-inflammatory substance may be released within the patient's body, when the carrier including the releasing element has been deposited within the patient's body.

The following list of aspects provides preferred embodiments of the present disclosure:
1. A medical device configured to be deposited at least partially within a patient's body via the patient's vagina for treating dysmenorrhea and/or endometriosis, the medical device including:
   at least one carrier,
   at least one anti-inflammatory substance which is at least partially attached to the carrier such that the anti-inflammatory substance is releasable within the patient's body, when the medical device is deposited within the patient's body.
2. The medical device according to aspect 1, further including at least one releasing element which is at least partially attached to the carrier, wherein the anti-inflammatory substance is attached to, preferably embedded at least partially within, the releasing element.
3. The medical device according to aspect 2, wherein the releasing element is at least partially, preferably completely, made of at least one biodegradable material.
4. The medical device according to any of the preceding aspects, wherein the at least one anti-inflammatory substance includes at least one non-hormonal and/or non-steroidal anti-inflammatory substance, preferably celecoxib and/or diclofenac.
5. The medical device according to any of the preceding aspects, wherein the at least one anti-inflammatory substance includes diclofenac and/or celecoxib.
6. The medical device according to any of the preceding aspects, wherein the medical device is configured to be deposited in at least a section of the patient's uterus.
7. The medical device according to any of the preceding aspects, wherein the medical device is free of hormonal substances.
8. The medical device according to any of the preceding aspects, wherein the medical device is configured to release the anti-inflammatory substance continuously at a rate larger than zero within the patient's body, when the medical device is deposited within the patient's body.
9. The medical device according to any of aspects 2 to 8, wherein the releasing element is configured to interact with an environment within the patient's body to trigger the release of the anti-inflammatory substance, preferably wherein the releasing element is configured to continuously release the anti-inflammatory substance thereafter.
10. The medical device according to any of aspects 2 to 9, wherein the releasing element is configured to at least partially disintegrate within the patient's body, when the medical device is deposited within the patient's body, wherein the anti-inflammatory substance is at least partially embedded in the releasing element such that the anti-inflammatory substance is released into the patient's body as the releasing element disintegrates, when the medical device is deposited within the patient's body.
11. The medical device according to any of aspects 2 to 10, wherein the anti-inflammatory substance is dispersed within the releasing element.
12. The medical device according to any of aspects 2 to 11, wherein the releasing element is configured to at least partially degrade within the patient's body at a preferably constant rate, when the medical device is deposited within the patient's body, such that the anti-inflammatory substance is released into the patient's body as the releasing element degrades.
13. The medical device according to any of aspects 2 to 12, wherein the releasing element, including the anti-inflammatory substance, is configured as a solid component, wherein the releasing element is at least partially hollow, preferably wherein the releasing element is configured as a tube configured to at least partially surround at least a section of the carrier.
14. The medical device according to any of aspects 2 to 13, wherein the carrier includes at least one receiving section which is configured to securely receive the releasing element, preferably wherein the receiving section is configured as an elongated member which is configured to be received at least partially within the releasing element.
15. The medical device according to aspect 14, wherein the receiving section has a diameter of no more than 2.4 mm, preferably no more than 2.2 mm, more preferably no more than 2 mm, more preferably no more than 1.8 mm, more preferably no more than 1.6 mm, more preferably no more than 1.4 mm, more preferably no more than 1.2 mm, more preferably no more than 1 mm.
16. The medical device according to any of the preceding aspects, wherein the medical device includes at least one securing mechanism configured to secure the medical device in at least a section of the patient's uterus.
17. The medical device according to aspect 16, wherein the securing mechanism includes at least one arm which is configured to engage with a wall of the patient's uterus to secure the medical device within the patient's uterus.
18. The medical device according to aspect 16 or 17, wherein the securing mechanism includes at least two arms which are configured to engage with substantially opposite sides of the patient's uterus to secure the medical device within the patient's uterus.
19. The medical device according to aspect 18, wherein the arms are attached to the receiving section such that the arms and the receiving section are configured substantially in a Y-form or a T-form.
20. The medical device according to any of the preceding aspects, wherein the anti-inflammatory substance is at least partially attached to at least one of the arms, preferably both arms.
21. The medical device according to any of the preceding aspects, wherein the medical device is configured to release the anti-inflammatory substance, preferably continuously, for at least 10 months, more preferably at least 11 months, more preferably at least 12 months, more preferably at least 13 months, more preferably at least 14 months, more preferably at least 15 months, more preferably at least 16 months, more preferably at least 17 months, more preferably at least 18 months, more preferably at least 19 months, more preferably at least 20 months, more preferably at least 21 months, more preferably at least 22 months, more preferably at least 23 months, more preferably at least 24 months within the patient's body.
22. The medical device according to any of the preceding aspects, wherein the medical device is configured to release the anti-inflammatory substance, preferably continuously, at a daily release rate of less than 3 mg per day, more preferably less than 2.8 mg per day, more preferably less than 2.6 mg per day, more preferably less than 2.4 mg per day, more preferably less than 2.2 mg per day, more preferably less than 2 mg per day, more preferably less than 1.8 mg per day, more preferably less than 1.6 mg per day, more preferably less than 1.4 mg per day, more preferably less than 1.2 mg per day, more preferably less than 1 mg per day.
23. The medical device according to any of the preceding aspects, wherein the medical device is configured to release the anti-inflammatory substance at a daily release rate in a range from 0.2 mg to 1 mg per day, preferably from 0.4 mg to 1 mg per day, more preferably from 0.6 mg to 1 mg per day, more preferably from 0.8 mg to 1 mg per day
24. The medical device according to any of the preceding aspects, wherein the medical device is configured to release the anti-inflammatory substance at a daily release rate with a day-to-day variance of no more than 0.3 mg per day, preferably no more than 0.2 mg, more preferably no more than 0.1 mg.
25. The medical device according to any of the preceding aspects, wherein the medical device is provided with at least 100 mg, preferably at least 150 mg, more preferably at least 200 mg, more preferably at least 250 mg, more preferably at least 300 mg, more preferably at least 350 mg, more preferably at least 400 mg, more preferably at least 450 mg, more preferably at least 500 mg of the anti-inflammatory substance.
26. The medical device according to any of the preceding aspects, further including a withdrawal apparatus configured to enable the medical device to be withdrawn from the patient's body.
27. The medical device according to any of the preceding aspects, further including at least one hemostatic agent, which is preferably attached to the carrier.
28. A kit including:
   at least one carrier configured to be deposited at least partially within a patient's body via the patient's vagina,
   at least one anti-inflammatory substance;
   wherein the anti-inflammatory substance is configured to be at least partially attachable to the carrier such that the anti-inflammatory substance is releasable within the patient's body, when the anti-inflammatory substance is attached to the carrier and the carrier including the releasing element is deposited within the patient's body.
29. The kit according to aspect 28, further including at least one releasing element, wherein the anti-inflammatory substance is attached to, preferably embedded at least partially within, the releasing element, wherein the releasing element is configured to be at least partially attachable to the carrier such that the anti-inflammatory substance is releasable within the patient's body, when the releasing element is attached to the carrier and the carrier is deposited within the patient's body
30. The kit according to aspect 29, wherein the releasing element is at least partially, preferably completely, made of at least one biodegradable material.
31. A method for treating at least one ailment of a female reproductive system of a patient, preferably dysmenorrhea and/or endometriosis, preferably by means of the medical device according to any of aspects 1 to 27, the method including:
   depositing a medical device which includes at least one carrier and at least one anti-inflammatory substance, which is at least partially attached to the carrier, at least partially within the patient's body via the patient's vagina, and
   releasing the anti-inflammatory substance within the patient's body, when the medical device has been deposited within the patient's body.
32. The method according to aspect 31, wherein the anti-inflammatory substance is attached to, preferably embedded at least partially within, at least one releasing element, wherein the releasing element is at least partially attached to the carrier.
33. The method according to aspect 32, wherein the releasing element is at least partially, preferably completely, made of at least one biodegradable material.
34. An anti-inflammatory substance, preferably non-steroidal anti-inflammatory substance and/or non-hormonal anti-inflammatory substance, preferably diclofenac and/or celecoxib, for use in treating dysmenorrhea and/or endometriosis, preferably by means of the medical device according to any of aspects 1 to 27, wherein the anti-inflammatory substance is embedded in at least one releasing element made of at least one biodegradable material, the releasing element being at least partially attached to at least one carrier, wherein the carrier, including the releasing element and the embedded anti-inflammatory substance, is deposited at least partially within the patient's body via the patient's vagina, and wherein the anti-inflammatory substance is released within the patient's body, when the carrier including the releasing element has been deposited within the patient's body.

Preferred embodiments of the present invention are further elucidated below with reference to the figures. The described embodiments are merely exemplary and do not limit the present invention.
- Fig. 1: shows, in a schematic illustration, a medical device according to an embodiment of the present disclosure;
- Fig. 2: shows, in a schematic illustration, a medical device according to a further embodiment of the present disclosure.

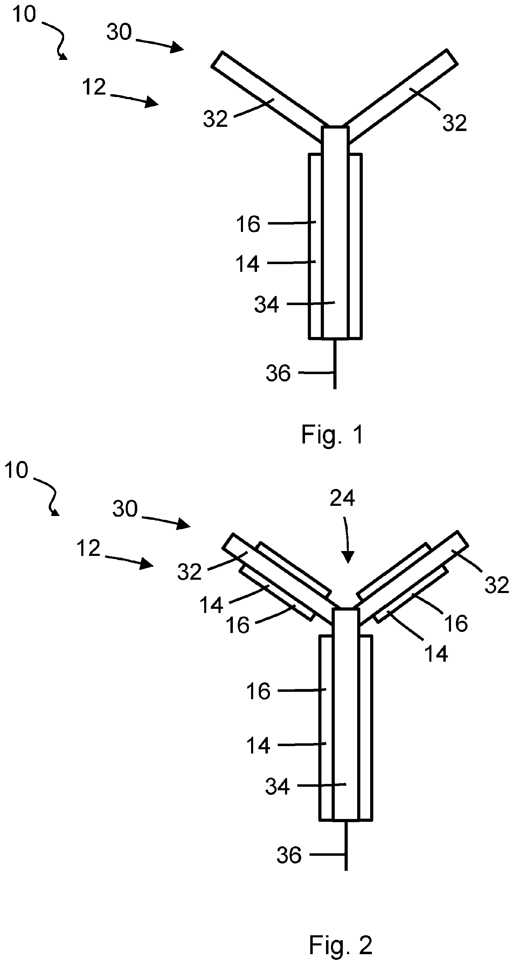

Fig. 1 schematically shows a medical device 10 which may be configured to be deposited at least partially within a patient's body via the patient's vagina for treating dysmenorrhea and/or endometriosis. The medical device 10 may include at least one carrier 12 and at least one releasing element 14 which may be made of at least one biodegradable material, preferably at least one biodegradable biopolymer. The medical device 10 may further include at least one anti-inflammatory substance 16 which may be at least partially embedded or trapped in the releasing element 14. The releasing element 14 may be at least partially attached to the carrier 12 such that the anti-inflammatory substance 16 is releasable within the patient's body, when the medical device 10 is deposited within the patient's body.

The releasing element 14 may be configured to at least partially disintegrate/ degrade within the patient's body, when the medical device 10 is deposited within the patient's body. The anti-inflammatory substance 16 may be released in the patient's body as the releasing element 14 disintegrates/degrades, when the medical device 10 is deposited within the patient's body.

Preferably, the releasing element 14, including the anti-inflammatory substance 16, is configured in a solid aggregate state. The releasing element 14 may include at least one lumen (not shown in the figures) which is configured to receive at least a section of the carrier 12 to secure the releasing element 14 to the carrier 12. Preferably, the releasing element 14 may be configured as tube configured to, at least partially, surround, at least, a section of the carrier 12.

The at least one anti-inflammatory substance 16 includes at least one non-hormonal and/or non-steroidal anti-inflammatory substance, preferably celecoxib and/or diclofenac. However, a number of alternative anti-inflammatory substances 16 may also be employed. For instance, alternative anti-inflammatory substances may include one or more of ibuprofen, ketoprofen, mefenamic acid, naproxen, piroxicam, and indomethacin. Alternative non-hormonal anti-inflammatory substances may include glyceryl trinitrate which may require even lower concentrations and/or release rates than other anti-inflammatory substances, in particular non-hormonal anti-inflammatory substances.

An advantage of the medical device 10 described herein may be that the release of the treatment substance, i.e., the anti-inflammatory substance 16, may be controlled relatively precisely, e.g., which may allow the dose and/or release rate of the anti-inflammatory substance to be reduced and/or held relatively consistent/continuous. For instance, the release rate of the anti-inflammatory substance 16 may be controlled/adjusted by controlling/adjusting a rate of disintegration/degradation of the releasing element 14.

The medical device 10 may be configured to release the anti-inflammatory substance 16 continuously at a rate larger than zero within the patient's body, when the medical device 10 is deposited within the patient's body. The medical device 10 may be configured to release the anti-inflammatory substance 16, preferably continuously, at a daily release rate of less than 3 mg per day, more preferably less than 2 mg per day, more preferably less than 1 mg per day.

The medical device 10 may include at least one securing mechanism 30 configured to secure the medical device 10 within the patient's body, preferably in at least a section of the patient's uterus, more specifically in a patient's uterine cavity. The securing mechanism 30 may include at least one arm 32 which is configured to engage with a wall of the patient's uterus to secure the medical device 10 within the patient's uterus. Alternatively, the securing mechanism 30 may include a plurality of arms, e.g., at least two arms 32, as shown in Figs. 1 and 2. The arms 32 may be configured to engage with substantially opposite sides of the patient's uterus to secure the medical device 10 within the patient's uterus.

The carrier 12 may include at least one receiving section 34 which is configured to securely receive the releasing element 14. For instance, the receiving section 34 may be configured as an elongated member which is configured to be received at least partially within the releasing element 14.

The arms 32 may be attached to the receiving section 34 such that the arms 32 and the receiving section 34 are configured substantially in a Y-form or a T-form.

The medical device 10 may further include a withdrawal apparatus 36 configured to enable the medical device 10 to be withdrawn from the patient's body. The withdrawal apparatus 36 may be configured as one or more graspable elements which can be grasped by a human, e.g., the patient and/or medical personnel. For instance, the withdrawal apparatus 36 may include one or more elongated elements as shown in Figs. 1 and 2, e.g., one or more strings, which extend towards or into the patient's cervical canal and/or vagina, when the medical device 10 is employed in an operational state within the patient's body.

The anti-inflammatory substance 16 may also be arranged at, and optionally attached to, one or more of the arms 32, as shown in Fig. 2. For instance, the releasing element 14 may extend across at least a section of one or more of the arms 32. In this case, preferably, the medical device 10 may include a plurality of releasing elements 14, e.g., at least one first releasing element 14 may be arranged at, and optionally attached to, one or more of the arms 32 and at least one second releasing element 14 may be arranged at, and optionally attached to, the receiving section 34.

## Claims

1. A medical device (10) configured to be deposited at least partially within a patient's body via the patient's vagina for treating dysmenorrhea and/or endometriosis, the medical device (10) including:
at least one carrier (12),
at least one releasing element (14) which is at least partially attached to the carrier (12) and is made of at least one biodegradable material; and
at least one anti-inflammatory substance (16) which is embedded in the releasing element (14) such that the anti-inflammatory substance (16) is releasable within the patient's body, when the medical device (10) is deposited within the patient's body.

2. The medical device (10) according to claim 1, wherein the at least one anti-inflammatory substance (16) includes at least one non-hormonal and/or non-steroidal anti-inflammatory substance.

3. The medical device (10) according to claim 1 or 2, wherein the at least one anti-inflammatory substance (16) is celecoxib and/or diclofenac.

4. The medical device (10) according to any of the preceding claims, wherein the releasing element (14) is configured to at least partially disintegrate within the patient's body, when the medical device (10) is deposited within the patient's body, wherein the anti-inflammatory substance (16) is at least partially embedded in the releasing element (14) such that the anti-inflammatory substance (16) is released into the patient's body as the releasing element (14) disintegrates, when the medical device (10) is deposited within the patient's body.

5. The medical device (10) according to any of the preceding claims, wherein the releasing element (14) is configured to at least partially degrade within the patient's body at a preferably constant rate, when the medical device (10) is deposited within the patient's body, such that the anti-inflammatory substance (16) is released into the patient's body as the releasing element (14) degrades.

6. The medical device (10) according to any of the preceding claims, wherein the releasing element (14), including the anti-inflammatory substance (16), is configured as a solid component, wherein the releasing element (14) is at least partially hollow, preferably wherein the releasing element (14) is configured as a tube configured to at least partially surround at least a section the carrier (12).

7. The medical device (10) according to any of the preceding claims, wherein the medical device (10) includes at least one securing mechanism (30) configured to secure the medical device (10) in at least a section of the patient's uterus.

8. The medical device (10) according to claim 7, wherein the securing mechanism (30) includes at least two arms (32) which are configured to engage with substantially opposite sides of the patient's uterus to secure the medical device (10) within the patient's uterus.

9. The medical device (10) according to any of the preceding claims, wherein the releasing element (14) including the embedded anti-inflammatory substance (16) is at least partially attached to at least one of the arms, preferably both arms.

10. The medical device (10) according to any of the preceding claims, wherein the medical device (10) is configured to release the anti-inflammatory substance (16), preferably continuously, for at least 10 months, more preferably at least 11 months, more preferably at least 12 months, more preferably at least 13 months, more preferably at least 14 months, more preferably at least 15 months, more preferably at least 16 months, more preferably at least 17 months, more preferably at least 18 months, more preferably at least 19 months, more preferably at least 20 months, more preferably at least 21 months, more preferably at least 22 months, more preferably at least 23 months, more preferably at least 24 months within the patient's body.

11. The medical device (10) according to any of the preceding claims, wherein the medical device (10) is configured to release the anti-inflammatory substance (16), preferably continuously, at a daily release rate of less than 3 mg per day, more preferably less than 2.8 mg per day, more preferably less than 2.6 mg per day, more preferably less than 2.4 mg per day, more preferably less than 2.2 mg per day, more preferably less than 2 mg per day, more preferably less than 1.8 mg per day, more preferably less than 1.6 mg per day, more preferably less than 1.4 mg per day, more preferably less than 1.2 mg per day, more preferably less than 1 mg per day.

12. The medical device (10) according to any of the preceding claims, wherein the medical device (10) is configured to release the anti-inflammatory substance (16) at a daily release rate with a day-to-day variance of no more than 0.3 mg per day, preferably no more than 0.2 mg, more preferably no more than 0.1 mg.

13. A kit including:
at least one carrier (12) configured to be deposited at least partially within a patient's body via the patient's vagina,
at least one releasing element (14) made of at least one biodegradable material;
at least one anti-inflammatory substance (16) embedded in the releasing element (14);
wherein the releasing element (14) is configured to be at least partially attachable to the carrier (12) such that the anti-inflammatory substance (16) is releasable within the patient's body, when the releasing element (14) is attached to the carrier (12) and the carrier (12) including the releasing element (14) is deposited within the patient's body.

14. A non-steroidal and/or non-hormonal anti-inflammatory substance for use in treating dysmenorrhea and/or endometriosis, preferably by means of the medical device (10) according to any of claims 1 to 12, wherein the anti-inflammatory substance (16) is embedded in at least one releasing element (14) made of at least one biodegradable material, the releasing element (14) being at least partially attached to at least one carrier (12), wherein the carrier (12), including the releasing element (14) and the embedded anti-inflammatory substance (16), is deposited at least partially within the patient's body via the patient's vagina, and wherein the anti-inflammatory substance (16) is released within the patient's body, when the carrier (12) including the releasing element (14) has been deposited within the patient's body.

15. The non-steroidal and/or non-hormonal anti-inflammatory substance of claim 14, wherein the substance is diclofenac and/or celecoxib.
